# EUROPÄISCHE PATENTSCHRIFT

(11) **EP 3 160 936 B1**
(45) Veröffentlichungstag und Bekanntmachung des Hinweises auf die Patenterteilung: **13.10.2021**
(21) Anmeldenummer: 15731295.0
(22) Anmeldetag: 22.06.2015
(51) Int. Cl.: C07C 209/78

(54) **VERFAHREN ZUR HERSTELLUNG VON DI- UND POLYAMINEN DER DIPHENYLMETHANREIHE**
METHOD FOR PRODUCING DI- AND POLYAMINES OF THE DIPHENYLMETHANE SERIES
PROCÉDÉ DESTINÉ À LA FABRICATION DE DI- ET POLYAMINES DE LA SÉRIE DIPHÉNYLMÉTHANE

(30) Priorität: 24.06.2014 EP 14173582
(43) Veröffentlichungstag der Anmeldung: 03.05.2017
(73) Patentinhaber: Covestro Intellectual Property GmbH & Co. KG, 51373 Leverkusen (DE)
(72) Erfinder: KNAUF, Thomas, 41542 Dormagen (DE); WERSHOFEN, Stefan, 41065 Mönchengladbach (DE); GRUNER, Klaus-Gerd, 47239 Duisburg (DE); HARTJES, Volker, 47239 Duisburg (DE)
(74) Vertreter: Levpat
(86) Internationale Anmeldenummer: PCT/EP2015/063923
(87) Internationale Veröffentlichungsnummer: WO 2015/197520

(56) Entgegenhaltungen:
- EP-A1- 0 283 757
- EP-A1- 1 616 890
- EP-A1- 2 486 975
- None

## Beschreibung

Die Erfindung betrifft ein Verfahren zur Herstellung von Di- und Polyaminen der Diphenylmethanreihe (MDA) aus Anilin und Formaldehyd, bei dem während des Anfahrvorganges Sorge getragen wird, dass ein ausreichender Überschuss von Anilin gegenüber Formaldehyd gewährleistet ist, der mindestens 105 % des für die Zielrezeptur des zu produzierenden MDA angestrebten molaren Verhältnisses von Anilin zu Formaldehyd beträgt.

Die kontinuierliche oder teilweise diskontinuierliche Herstellung von MDA ist z. B. in EP 1 616 890 A1, US-A-5286760, EP-A-451442 und WO-A-99/40059 offenbart. Die saure Kondensation von aromatischen Aminen und Formaldehyd zu Di- und Polyaminen der Diphenylmethanreihe läuft in mehreren Reaktionsschritten ab.

Beim Aminalverfahren wird zunächst in Abwesenheit eines sauren Katalysators Formaldehyd mit Anilin zu sogenanntem Aminal kondensiert, wobei Wasser abgespalten wird. Danach erfolgt die Umlagerung zum MDA säurekatalysiert in einem ersten Schritt zu para- bzw. ortho-Aminobenzylanilin. Die Aminobenzylaniline lagern in einem zweiten Schritt zum MDA um. Hauptprodukte der säurekatalysierten Reaktion von Anilin und Formaldehyd sind das Diamin 4,4'-MDA, seine Stellungsisomere 2,4'-MDA und 2,2'-MDA sowie höhere Homologe.

Beim Neutralisationsverfahren werden in Anwesenheit eines sauren Katalysators Anilin und Formaldehyd direkt zu Aminobenzylanilinen umgesetzt, die anschließend weiter zu den zweikernigen MDA-Isomeren und höherkernigen MDA-Homologen reagieren.

Unabhängig von der Verfahrensvariante zur Herstellung des sauren Reaktionsgemisches wird dessen Aufarbeitung gemäß dem Stand der Technik durch Neutralisation mit einer Base eingeleitet. Die Neutralisation erfolgt üblicherweise bei Temperaturen von beispielsweise 90 °C bis 100 °C ohne Zusatz weiterer Substanzen. (H.J. Twitchett, Chem. Soc. Rev. 3(2), 223 (1974)). Sie kann aber auch auf einem anderen Temperaturniveau erfolgen, um z.B. den Abbau von störenden Nebenprodukten zu beschleunigen. Hydroxide der Alkali- und Erdalkalielemente sind als Basen geeignet. Vorzugsweise kommt wässrige NaOH zum Einsatz.

Im Anschluss an die Neutralisation wird in einem Trennbehälter die organische von der wässrigen Phase getrennt. Die nach Abtrennung der wässrigen Phase verbleibende, Roh-MDA enthaltende organische Phase wird weiteren Aufarbeitungsschritten unterzogen, wie z.B. einer Wäsche mit Wasser (Basenwäsche), um Restsalze aus dem Roh-MDA zu waschen. Abschließend wird das so gereinigte Roh-MDA von überschüssigem Anilin, Wasser und anderen im Gemisch vorhandenen Stoffen (z.B. weiteren Lösungsmitteln) durch geeignete Verfahren wie z.B. Destillation, Extraktion oder Kristallisation befreit. Die nach dem Stand der Technik übliche Aufarbeitung ist beispielsweise offenbart in EP 1 652 835 A1, Seite 3, Zeile 58 bis Seite 4, Zeile 13 oder EP 2 103 595 A1, Seite 7, Zeile 21 bis 37.

EP 2 486 975 A1 offenbart die Anwendung eines speziellen Mischer-Reaktors in der Herstellung von MDA. Es wird beschrieben, dass ein lokaler Formaldehydüberschuss zur Bildung netzartiger Polymere führen kann. Der Patentanmeldung sind jedoch keinerlei Details zur Ausgestaltung des Anfahrens der Reaktion, also der Aufnahme oder Wiederaufnahme des Verfahrens nach einer Unterbrechung, zu entnehmen. Insbesondere lehrt die Patentanmeldung nicht, dass *während des Anfahrvorgangs* das "A/F-Verhältnis" (das molare Verhältnis von Anilin zu Formaldehyd) **über** dem A/F-Verhältnis *während des Regelbetriebs* liegen sollte.

EP 1 616 890 A1 lehrt, dass Anilin und Formaldehyd zunächst in Abwesenheit des sauren Katalysators zu Aminal umgesetzt werden, und das Aminal anschließend mit saurem Katalysator versetzt wird, und es bei Temperaturen von 20 °C bis 100 °C und bei Wassergehalten des so erhaltenen sauren Reaktionsgemisches von 0 bis 20 Gewichtsprozent weiter umgesetzt wird. Insbesondere wird nach der Kondensation von Formaldehyd und Anilin zum Aminal zunächst das Wasser zumindest teilweise aus dem Aminal entfernt, wobei man einen Wassergehalt von 0 bis 5 Gewichtsprozent im Aminal einstellt, und anschließend das Aminal mit saurem Katalysator versetzt, und es bei Temperaturen von 20 °C bis 100 °C und bei Wassergehalten des so erhaltenen sauren Reaktionsgemisches von 0 bis 20 Gewichtsprozent weiter umsetzt. So können Gemische der Di- und Polyamine der Diphenylmethanreihe bei Protonierungsgraden von < 15 %, bevorzugt 4 % bis 14 %, besonders bevorzugt 5 % bis 13 %, hergestellt werden. Als Protonierungsgrad wird dabei bei einprotonigen sauren Katalysatoren (wie Salzsäure) das molare Verhältnis aus der Menge an eingesetztem sauren Katalysator und der im Reaktionsgemisch vorhandenen molaren Menge an Aminfunktionen bezeichnet. Die Patentanmeldung geht in keiner Weise auf die Vorgehensweise während des Anfahrvorgangs einer großtechnischen Produktionsanlage ein. Die enthaltenen Beispiele sind Laborexperimente. Insbesondere lehrt diese Patentanmeldung nicht, dass *während des Anfahrvorgangs* das A/F-Verhältnis **über** dem A/F-Verhältnis *während des Regelbetriebs* liegen sollte.

EP 0 283 757 A1 befasst sich ebenfalls mit der Herstellung von MDA. Das beschriebene Verfahren ist gekennzeichnet durch die Zufuhr anilinfreien MDA's zu durch Kondensation von Anilin und Formaldehyd gebildeten Aminobenzylaminen vor deren durch Wärme induzierter Umlagerungsreaktion. In Beispiel 2 wird eine Verfahrensweise beschrieben, bei der ein geringer Teil des gebildeten MDA in die Umlagerungsreaktion zurückgeführt wird (vgl. auch Patentansprüche 6 und 8). Mit anderen Worten: beschrieben wird die Verschaltung einer MDA-Anlage *im kontinuierlichen Regelbetrieb.* Details zur Vorgehensweise beim Anfahren einer MDA-Anlage werden nicht beschrieben; insbesondere gibt es keine Angaben zum A/F-Verhältnis während des Anfahrens im Vergleich zum A/F-Verhältnis während der Reaktion.

WO-A-99/40059 lehrt, dass man zur Herstellung von Metyhlendi(phenylamin) durch Umsetzung von Anilin mit Formaldehyd in Gegenwart saurer Katalysatoren in einem halbkontinuierlichen Verfahren Anilin und gegebenenfalls sauren Katalysator vorlegt, Formaldehyd und gegebenenfalls sauren Katalysator durch ein Mischorgan in einen Kreislauf, in dem Anilin, gegebenenfalls saurer Katalysator und gegebenenfalls bereits zugegebenes Formaldehyd kreisläufig bewegt werden, einspeist und nach Einspeisung von mindestens 50 % der gesamt einzuspeisenden Formaldehydmenge die Reaktionsmischung auf eine Temperatur von grösser 75 °C temperiert. Die Zugabe bis zu einer Menge von mindestens 50 % der gesamt einzuspeisenden Formaldehydmenge erfolgt bei einer Temperatur des Reaktionsgemisches im Kreislauf von 20 °C bis 75 °C.

Keines der vorgenannten Dokumente des Standes der Technik legt es nahe, beim Anfahren der Reaktion zur Herstellung von MDA ein A/F-Verhältnis einzusetzen, das von dem während des Regelbetriebs abweicht. So ist es im Stand der Technik durchaus üblich, im Regelbetrieb A/F-Verhältnisse einzusetzen, die über dem durch die Stöchiometrie der Reaktion vorgegebenen (2 : 1) liegen. Der Stand der Technik legt jedoch in keiner Weise nahe, während des Anfahrens *noch größere* A/F-Verhältnisse einzuhalten.

Die Güte eines Verfahrens zur Herstellung von MDA ist einerseits definiert durch den Gehalt des Produktes an unerwünschten Nebenprodukten der Reaktion. Andererseits ist die Güte eines Verfahrens dadurch definiert, dass der gesamte Prozess von Anfahren, normaler Produktion bis zum Abfahren des Prozesses ohne technischen Produktionsausfall oder Problemen, die zu einem Eingriff in den Prozess nötigen, betrieben werden kann, und dass es nicht zu Verlusten an Einsatzstoffen, Zwischenprodukten oder an Endprodukt kommt.

Solche Probleme können z.B. beim Anfahren der Aminalreaktion auftreten. Derartige Probleme können z.B. sein, dass es zur Entstehung von hochmolekularen Feststoffen kommt, die zu Anbackungen und Verblockungen an der Ausrüstung (Aminalkessel, -kühler und -abscheider und Leitungen) führen.

Den beschriebenen Verfahren des Standes der Technik gelingt es zwar, mit hoher Ausbeute MDA herzustellen, ohne dass es bei den Endprodukten zu einer Qualitätseinbuße kommt, jedoch werden nur Verfahren beschrieben, die sich im Normalbetrieb befinden. Anfahrprozesse bis zum Erreichen eines stabilen Betriebszustandes bei der angestrebten Soll-Last (sogenannte "Anfahrzeit") werden nicht berücksichtigt. Ein semikontinuierlich oder kontinuierlich betriebener industrieller Prozess kann, ausgehend von einer nicht in Betrieb befindlichen Produktionsanlage (z. B. nach einem wartungsbedingtem Stillstand), nicht augenblicklich wieder auf die Prozessparameter vor dem Produktionsstillstand hochgefahren werden. Edukte und Apparaturen müssen aufgeheizt werden, Apparate müssen ggf. inertisiert werden, die Belastung der Apparate mit den Edukten wird allmählich auf den angestrebten Soll-Wert gesteigert. An- und Abfahrzeiten treten im industriellen Alltag häufig auf und sind nicht zwangsläufig mit einem Öffnen oder einem anderen mechanischen Eingriff in einen Reaktor oder einen anderen Apparat der Anlage verbunden, sondern können auch mit dem Abstellen und erneuten Starten der Produktionsanlage aus diversen anderen Gründen in Verbindung stehen, wie z.B. Rohstoffmangel. Diese Anfahrzeiten zeichnen sich in der Praxis dadurch aus, dass es zu Abweichungen im angestrebten molaren Mengenverhältnis von Anilin gegenüber Formalin kommen kann.

Wünschenswert wäre daher ein verbessertes Verfahren zur Herstellung von Di- und Polyaminen der Diphenylmethanreihe, wobei das Augenmerk auf den Zeitraum des Anfahrens der Aminalreaktion gelegt wird. Die vorliegende Erfindung hat sich die Aufgabe gestellt, ein solches Verfahren bereitzustellen.

Erfindungsgemäß gelöst wird diese Aufgabe durch ein Verfahren zur Herstellung von Di- und Polyaminen der Diphenylmethanreihe (MDA) durch Umsetzung von Anilin (1) und Formaldehyd (2) bei einem angestrebten molaren Verhältnis von Anilin (1) zu Formaldehyd (2) von A/F_{Ziel}, welches bevorzugt einen Wert von 1,5 bis 20, besonders bevorzugt von 1,5 bis 15, ganz besonders bevorzugt von 1,5 bis 10 und außerordentlich ganz besonders bevorzugt von 1,5 bis 6, aufweist , umfassend die Schritte:

### Entweder gemäß einer Variante A)

A.I) Reaktion von Anilin (1) und Formaldehyd (2) in einem Reaktor in Abwesenheit eines sauren Katalysators (3) unter Bildung eines Aminals, wobei Anilin (1) und Formaldehyd (2) in den Reaktor eingetragen werden, und anschließende Trennung des erhaltenen Reaktionsgemisches in eine wässrige und eine organische, Aminal enthaltende Phase;
A.II) Reaktion zumindest eines Teils der in Schritt A.I) erhaltenen organischen, Aminal enthaltenden Phase in einem Reaktor mit Säure (3), wobei das Aminal zu Di- und Polyaminen der Diphenylmethanreihe reagiert;
   wobei die Schritte A.I) und A.II) kontinuierlich durchgeführt werden und wobei für die Aufnahme des Verfahrens und/oder die Wiederaufnahme des Verfahrens nach einer Unterbrechung zumindest des Schritts A.I) die folgenden Schritte durchlaufen werden:
   A.I.1) Eintragen von Anilin (1), beginnend bei dem Zeitpunkt t₀, in den Reaktor aus Schritt A.I) mit einem Massenstrom m₁;
   A.I.2) Eintragen von Formaldehyd (2), beginnend bei einem Zeitpunkt t₁, in den Reaktor aus Schritt A.I) ausgehend von einem Massenstrom m₂ = 0 beim Zeitpunkt t₁ bis zu einem Massenstrom m₂ = m_{2,Soll} beim Zeitpunkt t₂, wobei t₂ > t₁ > t₀ und wobei die Zeitspanne t₂ - t₁ bei > 0 Stunden und < 20 Stunden liegt, wobei der Zeitpunkt t₁ so gewählt wird, dass zu diesem Zeitpunkt der Reaktor zu 10 % bis 99 % seines maximalen Fassungsvermögens mit Anilin befüllt ist;
   A.II.1) im Falle der Unterbrechung auch des Schritts A.II), Eintragen von Säure (3) in den Reaktor aus Schritt A.II) spätestens wenn, sobald oder nachdem, bevorzugt sobald- oder nachdem, besonders bevorzugt nachdem, erstmalig organische, Aminal enthaltende Phase in den Reaktor aus Schritt A.II) eingetragen wird;
      wobei das Eintragen von Anilin (1) in Schritt A.I.1) und das Eintragen von Formaldehyd (2) in Schritt A.I.2) derart geschehen, dass im Zeitraum von t₁ bis t₂ das momentane molare Verhältnis von
      dem Reaktor aus Schritt A.I) insgesamt zugegebenem Anilin (1)
         zu
      dem Reaktor aus Schritt A.I) insgesamt zugegebenem Formaldehyd (2),
      A/F_{mom.}, stets ≥ 2 und ≥ 1,05 · A/F_{Ziel} beträgt,
      wobei nach Erreichen des angestrebten Formaldehyd-Massenstroms m_{2,Soll} zum Zeitpunkt t₂ das molare Verhältnis von Anilin (1) zu Formaldehyd (2) auf A/F_{Ziel} eingestellt wird;
   oder gemäß einer Variante B)
B.I) Reaktion von Anilin (1) und Säure (3) in einem Reaktor unter Bildung eines Reaktionsgemisches, das das Aniliniumsalz der verwendeten Säure (3) enthält;
B.II) Reaktion zumindest eines Teils des in Schritt B.I) erhaltenen Reaktionsgemisches mit Formaldehyd (2) in einem Reaktor, wobei Di- und Polyamine der Diphenylmethanreihe entstehen;
   wobei die Schritte B.I) und B.II) kontinuierlich durchgeführt werden und wobei für die Aufnahme des Verfahrens und/oder die Wiederaufnahme des Verfahrens nach einer Unterbrechung der Schritte B.I) und B.II) folgende Schritte durchlaufen werden:
   B.I.1) Eintragen von Anilin (1), beginnend bei dem Zeitpunkt t₀, in den Reaktor aus Schritt B.I) mit einem Anilin-Massenstrom m₁;
   B.I.2) Eintragen von Säure (3) vor, gleichzeitig mit oder nach dem Eintragen von Anilin (1);
   B.II.1) Eintragen von Formaldehyd (2) in den Reaktor aus Schritt B.II), optional gemeinsam mit weiterem Anilin (1), optional gemeinsam mit weiterer Säure (3), beginnend bei einem Zeitpunkt t₁, ausgehend von einem Massenstrom m₂ = 0 beim Zeitpunkt t₁ bis zu einem Massenstrom m₂ = m_{2,Soll} beim Zeitpunkt t₂, wobei t₂ > t₁ > t₀ und wobei die Zeitspanne t₂ - t₁ bei > 0 Stunden und < 20 Stunden liegt, wobei der Zeitpunkt t₁ so gewählt wird, dass zu diesem Zeitpunkt der Reaktor zu 10 % bis 99 % seines maximalen Fassungsvermögens mit Anilin befüllt ist;
      wobei das Eintragen von Anilin (1) in Schritt B.I.1) sowie ggf. in Schritt B.II.1) und das Eintragen von Formaldehyd (2) in Schritt B.II.1) derart geschehen, dass im Zeitraum von t₁ bis t₂ das momentane molare Verhältnis von
      dem Reaktor aus Schritt B.I) insgesamt zugegebenem Anilin (1) und, sofern vorhanden, dem Reaktor aus Schritt B.II) insgesamt zugegebenem Anilin (1)
         zu
      dem Reaktor aus Schritt B.II) insgesamt zugegebenem Formaldehyd (2),
      A/F_{mom.}, stets ≥ 2 und ≥ 1,05 · A/F_{Ziel} beträgt,
      wobei nach Erreichen des angestrebten Formaldehyd-Massenstroms m_{2,Soll} zum Zeitpunkt t₂ das molare Verhältnis von Anilin (1) zu Formaldehyd (2) auf A/F_{Ziel} eingestellt wird.

Im Sinne der vorliegenden Erfindung werden unter *"Di- und Polyaminen der Diphenylmethanreihe"* Amine und Gemische von Aminen folgenden Typs verstanden:

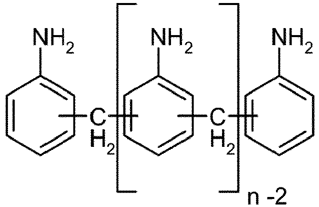

Dabei steht n für eine natürliche Zahl ≥ 2. Im Folgenden werden die Verbindungen dieses Typs, bei denen n = 2 ist, auch als Diamine der Diphenylmethanreihe oder Diaminodiphenylmethane (nachfolgend MMDA) bezeichnet. Verbindungen dieses Typs, bei denen n > 2 ist, werden im Rahmen dieser Erfindung auch als Polyamine der Diphenylmethanreihe oder Polyphenylenpolymethylenpolyamine (nachfolgend PMDA) bezeichnet. Gemische aus beiden Typen werden auch als Di- und Polyamine der Diphenylmethanreihe bezeichnet (nachfolgend MDA). Industriell werden die Di- und Polyamingemische überwiegend durch Phosgenierung zu den entsprechenden Di- und Polyisocyanaten der Diphenylmethanreihe umgesetzt.

In beiden Varianten können die *Reaktoren aus den Schritten I) bzw. II)* gleich oder verschieden sein. Das heißt, in Variante A) ist es sowohl möglich, das in Schritt A.I) gebildete Aminal im Reaktor zu belassen und die Säure zuzugeben, als auch das Aminal in einen anderen Reaktor zu überführen und dort dann die Säure (3) zuzugeben. In Variante B) ist es sowohl möglich, das in Schritt B.I) gebildete Reaktionsprodukt aus Anilin (1) und Säure (3) im Reaktor zu belassen und den Formaldehyd (2) zuzugeben, als auch das Reaktionsprodukt aus Anilin (1) und Säure (3) in einen anderen Reaktor zu überführen und dort dann den Formaldehyd (2) zuzugeben. Des Weiteren umfasst der Begriff *"ein Reaktor"* im Rahmen der vorliegenden Erfindung auch den Fall, dass eine Reaktorkaskade eingesetzt wird (mit anderen Worten, das Wort *"ein"* ist in diesem Zusammenhang als unbestimmter Artikel und nicht als Zahlwort aufzufassen).

In beiden Varianten werden die Schritte I) und II) kontinuierlich durchgeführt.

Der *Formaldehyd-Massenstrom m_{2,Soll}* bezeichnet den Formaldehyd-Massenstrom m₂ bei der angestrebten Produktionskapazität (der angestrebten Last, "Soll-Last"). Das *angestrebte molare Verhältnis von Anilin (1) zu Formaldehyd (2), A*/*F_{Ziel},* also das molare Verhältnis von Anilin zu Formaldehyd (CH₂O) in der Zielrezeptur, bestimmt die Größe der Einsatzströme (1) und (2) bei der Soll-Last (A/F_{Ziel} = [m_{1,Soll} / M₁] / [m_{2,Soll} / M₂], mit M₁ = Molmasse Anilin und M₂ = Molmasse Formaldehyd, CH₂O). Somit bezieht sich das A/F_{Ziel}-Verhältnis auf den Zeitraum nach Abschluss des Anfahrens der Produktion.

Das *momentane molare Verhältnis, A*/*F_{mom.},* im Zeitraum von t₁ bis t₂ ergibt sich **im Fall der Variante A)** in einfacher Weise aus den bekannten Einsatzströmen (1) und (2) in den Reaktor aus Schritt A.I) zu einem bestimmten Zeitpunkt t unter Berücksichtigung der bekannten Menge an Anilin (1), das bis zum Zeitpunkt t₁ bereits in den Reaktor aus Schritt A.I) eingetragen wurde. **Im Fall der Variante B)** ergibt sich das momentane molare Verhältnis, A/F_{mom.}, im Zeitraum von t₁ bis t₂ in analoger Weise aus den bekannten Einsatzströmen (1) und (2) in den Reaktor aus Schritt B.I) bzw. in den Reaktor aus Schritt B.II) zu einem bestimmten Zeitpunkt t unter Berücksichtigung der bekannten Menge an Anilin (1), das bis zum Zeitpunkt t₁ bereits in den Reaktor aus Schritt B.I) eingetragen wurde. Wird von der Möglichkeit Gebrauch gemacht, in Schritt B.II.1) weiteres Anilin in den Reaktor aus Schritt B.II) einzutragen, so wird dieses zum Zwecke der Bestimmung des momentanen molaren Verhältnisses, A/F_{mom.}, im Zeitraum von t₁ bis t₂ zum Anilin aus Schritt B.I.1) addiert. Wird solches in Schritt B.II.1) zugesetztes Anilin zuvor mit Säure vermischt, sodass es als Aniliniumsalz vorliegt, so ändert dies nichts an der Berechnung, da ein Mol Anilin mit einem Mol Säure zu einem Mol Aniliniumsalz reagiert. Für die Zwecke der Berechnung des momentanen molaren Verhältnisses A/F_{mom.} kann also so getan werden, als läge alles Anilin (1) in freier Form vor.

Das während des Anfahrens momentan vorliegende Verhältnis von Anilin (1) zu Formaldehyd (2), A/F_{mom.}, wird erfindungsgemäß stets so eingestellt, dass man sich dem Zielwert A/F_{Ziel}, grundsätzlich *von oben* nähert und nicht von unten. Das bedeutet, dass beispielsweise bei einem angestrebten A/F_{Ziel}-Verhältnis von 2,0 tatsächlich während des Zeitraumes von t₁ bis t₂ ein momentanes molares Verhältnis von Anilin (1) zu Formaldehyd (2), A/F_{mom.}, von mindestens 2,1 eingehalten wird. Nach Erreichen der angestrebten Last, d. h. nach Erreichen des angestrebten Formaldehyd-Massenstroms m_{2,Soll} zum Zeitpunkt t₂ wird das molare Verhältnis von Anilin (1) zu Formaldehyd (2) dann auf A/F_{Ziel}, im gewählten Beispiel also auf 2,0, eingestellt. Theoretisch reagieren zwei Mol Anilin mit einem Mol Formaldehyd zu einem Mol Diaminodiphenylmethan, sodass dieses Beispiel den Fall der stöchiometrischen Fahrweise beschreibt.

Im realen System werden neben den isomeren Diaminodiphenylmethanen bei einem A/F_{Ziel}-Verhältnis von 2,0 in der Zielrezeptur immer auch höhere Homologe gebildet, sodass ein Teil des eingesetzten Anilins zum Ende der Reaktion unverändert im resultierenden Reaktionsgemisch vorliegt.

Ausführungsformen des erfindungsgemäßen Verfahrens werden nachfolgend beschrieben. Sie können beliebig miteinander kombiniert werden, sofern sich aus dem Kontext nicht eindeutig das Gegenteil ergibt.

Wenn eine Produktionsanlage zur Herstellung von MDA bei einer Soll-Belastung m_{2,Soll} von x [kg(Formaldehyd)/h] betrieben werden soll, so kann diese Soll-Belastung erreicht werden, indem zu Beginn der Reaktion von Anilin mit Formaldehyd die Belastung m_{2,Soll} zunächst auf einen Wert von z. B. 0,25 x eingestellt und die Belastung dann über die Zwischenstufen m₂ = 0,50 x und m₂ = 0,75 x innerhalb einer Zeit t auf den Wert m₂ = x = m_{2,Soll} gesteigert wird.

Das Anfahren der MDA-Herstellung gelingt vor allen Dingen dann problemlos, wenn die Laststeigerung von einem Startwert m₂ = 0,0 x bis m₂ = x kontinuierlich und möglichst linear (mit Stufen oder stufenlos) zügig durchgeführt werden kann unter Beachtung aller betriebsrelevanten Parameter. Um einer schlechten Vermischung von Amin mit Formaldehyd im Reaktionsraum entgegenzuwirken und auch um keine Produktivität zu verlieren, sollte die Last unverzüglich und stufenlos auf mindestens 30 % der Ziellast (d. h. in der Regel die Nennlast; die Ziellast kann aber auch, z. B. bei geringer Nachfrage, von der Nennlast abweichen) der Reaktionsstraße erhöht werden. Sollte z. B. aus technischen Gründen oder wegen geringer Nachfrage an Produkt die angestrebte Ziellast geringer als die Nennlast sein, z. B. Halblastfahrweise, so wird analog verfahren.

Die Last wird bevorzugt unverzüglich auf 30 % bis 95 %, ganz bevorzugt auf 35 % bis 80 % und ganz besonders bevorzugt auf 40 % bis 60 % erhöht. Das Anfahrprocedere bis zum Erreichen von mindestens 30 % der Nennlast ist in einer Anfahrzeit von kleiner 20 Stunden, bevorzugt kleiner 10 Stunden und besonders bevorzugt kleiner 5 Stunden durchzuführen.

Dieses Beispiel steht natürlich nur exemplarisch für eine Vielzahl möglicher Anfahrprozeduren, deren genaue Ausgestaltung von den konkreten Gegebenheiten einer Produktionsanlage abhängt und daher nicht verallgemeinert werden kann. Ein gemeinsames Merkmal aller denkbaren Anfahrprozeduren ist jedoch, dass erst nach Verstreichen eines Zeitraums die angestrebte Soll-Belastung von x erreicht wird. Dieser Zeitraum wird als Anfahrzeit bezeichnet. Mit Anfahren ist insbesondere das Wieder-Anfahren der Anlage nach einem Kurzstillstand oder einem geplanten Stillstand gemeint.

Sollten zwei oder mehr MDA-Reaktorlinien parallel betrieben werden, dann kann, muss aber nicht, mit einer Reaktorlinie gestartet und die anderen Reaktorlinien nacheinander angefahren werden. Wenn die Nebensysteme so dimensioniert sind, dass sie z. B. das überschüssige Anilin, Reaktionswasser und das Wasser, das über wässriges Anilin und Formaldehyd in den Prozess eingebracht wird, beim Anfahren der Anlage unproblematisch aufnehmen und weiterverarbeiten können, dann ist es möglich, alle MDA-Reaktorlinien zeitnah anzufahren.

Erfindungsgemäß liegt in beiden Varianten A) und B) die Zeitspanne t₂ - t₁ bei > 0 Stunden und < 20 Stunden, bevorzugt < 10 Stunden und besonders bevorzugt bei < 5 Stunden.

In einer weiteren Ausführungsform des erfindungsgemäßen Verfahrens beträgt in beiden Varianten A) und B) in Schritt I) der Massenstrom m₁ ≥ 1000 kg/Stunde. Vorzugsweise beträgt dieser Massenstrom ≥ 2000 kg/Stunde bis ≤ 200000 kg/Stunde, mehr bevorzugt ≥ 3000 kg/Stunde bis ≤ 100000 kg/Stunde.

In einer weiteren Ausführungsform des erfindungsgemäßen Verfahrens beträgt in beiden Varianten A) und B) in Schritt I) der Massenstrom m_{2,Soll} ≥ 300 kg/Stunde. Vorzugsweise beträgt dieser Massenstrom ≥ 400 kg/Stunde bis ≤ 100000 kg/Stunde, mehr bevorzugt ≥ 500 kg/Stunde bis ≤ 50000 kg/Stunde.

Der eingesetzte Formaldehyd (2) kann in beiden Varianten aus allen bekannten Produktionsverfahren für Formaldehyd stammen. Lediglich beispielhaft sei das Silberkontakt-Verfahren erwähnt.

Erfindungsgemäß wird in beiden Varianten A) und B) der Zeitpunkt t₁ so gewählt, dass zu diesem Zeitpunkt der jeweilige Reaktor (das heißt der Reaktor aus Schritt A.I) bzw. aus Schritt B.II)) zu 10 % bis 99 %, bevorzugt 10 % bis 90 %, besonders bevorzugt 20 % bis 80 % seines Fassungsvermögens (bezogen auf das zur Verfügung stehende Innenvolumen des Reaktors) mit Anilin (1) befüllt ist.

Die Verfahrensführungen der Varianten A) und B) im Normalbetrieb bis zum Erhalt des Rohprodukts werden im Folgenden näher beschrieben:
Die Herstellung der rohen Di- und Polyamine der Diphenylmethanreihe nach **Variante A)** lässt sich exemplarisch wie folgt zusammenfassen:
a) Kernvorgang des Schritts A.I): Anilin und Formaldehyd werden in einem Reaktor (dem sog. "Aminalreaktor") in Abwesenheit eines sauren Katalysators zu Aminal und Wasser kondensiert, und das dabei entstehende Aminal wird aus dem Aminalreaktor ausgetragen, und
b) Wasser aus Schritt a), das im Wesentlichen aus Kondensationswasser der Aminalreaktion und Wasser aus dem Einsatzstoff Formaldehyd herrührt, wird zumindest teilweise aus dem Reaktionsgemisch der Aminalreaktion als wässrige Phase abgetrennt, und
c) Kernvorgang des Schritts A.II): das Aminal aus Schritt b) wird säurekatalysiert zu MDA umgelagert.

Die Kondensation von Anilin und Formaldehyd in **Schritt a)** kann nach einem Verfahren nach dem Stand der Technik durchgeführt werden. Dabei werden normalerweise Anilin und wässrige Formaldehydlösung bei Molverhältnissen im Bereich von 1,5 bis 20, bevorzugt 1,5 bis 10 und besonders bevorzugt 1,5 bis 6 bei Temperaturen von 20 °C bis 120 °C, bevorzugt 40 °C bis 110 °C und besonders bevorzugt 60 °C bis 100 °C zu Aminal und Wasser kondensiert. Der Reaktor des Schritts A.I wird bei Normaldruck oder im Überdruck betrieben. Bevorzugt liegt ein Druck von 1,05 bis 5 bar absolut, ganz bevorzugt von 1,1 bis 3 bar und ganz besonders bevorzugt von 1,2 bar bis 2 bar absolut vor. Die Druckhaltung erfolgt durch Druckregelungsventile, oder indem man die Abgassysteme von Aminalreaktor und dem Überlauf des Aminalabscheiders verbindet. Der Aminalabscheider und der Ablauf der wässrigen Phase sind vorzugsweise beheizt, um Anbackungen zu verhindern. Geeignete Anilinqualitäten sind z.B. beschrieben in EP 1 257 522 B1, EP 2 103 595 A1 und EP 1 813 598 B1. Es werden bevorzugt technische Qualitäten an Formalin (wässrige Lösung von Formaldehyd) mit 30 Massen % bis 50 Massen % Formaldehyd in Wasser eingesetzt. Jedoch sind auch Formaldehydlösungen mit niedrigeren oder höheren Konzentrationen oder auch der Einsatz gasförmigen Formaldehyds denkbar.

Zweckmäßigerweise sind der Reaktor des Schritts A.I) (auch als "Aminalreaktor" bezeichnet) und der Reaktor des Schritts A.II) (auch als "Umlagerungsreaktor" bezeichnet) voneinander verschieden. Es ist aber nicht ausgeschlossen, die Schritte A.I) und A.II) in demselben Reaktor durchzuführen.

Zunächst kann "Einsatzanilin" in den Aminalreaktor bei Temperaturen von 10 °C bis 60 °C vorgelegt werden. Auch in dem Aminalabscheider wird eine gewisse Menge Anilin vorgelegt, um den Pumpenschutz für die Aminalpumpe, die das Aminal zum Umlagerungsreaktor pumpt, zu gewährleisten. Das Einsatzanilin setzt sich aus frischem Anilin und gegebenenfalls Anilin aus der MDA-Destillation (weiter unten näher beschrieben; siehe Schritt h)) und gegebenenfalls Anilin aus der Abwasseraufbereitung zusammen.

Dann erfolgt beispielsweise bei schon laufender Anilindosierung die Zugabe des Formalins in das gut gerührte, vorgelegte Anilin, wobei die gesamte Anlage von Eduktströmen bis Produktabnahme betriebsbereit sein sollte. Der Aminalreaktor ist mit einem Wärmeaustauscher ausgerüstet, um die entstehende Reaktionswärme abzuführen. Alternativ kann auch das Einsatzanilin entsprechend abgekühlt werden. Theoretisch könnte auch gekühltes Formalin zur Aufnahme der Reaktionswärme genutzt werden. Zu Beginn der Eindosierung von Formalin in den Reaktionsraum liegt ein "unendlicher" Überschuss an Anilin vor.

Bei niedrigen A/F_{Ziel}-Verhältnissen besteht im Aminalabscheider die Gefahr der Ablagerung von Feststoffen ("Aminalfeststoffe"). Durch einen "Formalinsplit", bei dem nur ein Teil des zur Erreichung des A/F_{Ziel}-Wertes erforderlichen Formalins in der Aminalreaktion eingesetzt wird und das restliche Formalin unmittelbar vor, zeitgleich mit oder nach der Säurezugabe dem Reaktionsgemisch zudosiert wird, kann im Aminalbereich mit einem ausreichend hohen molaren Verhältnis von Anilin zu Formaldehyd gearbeitet werden, um die Feststoffbildung zu vermeiden. Zusätzlich kann in einem Bereich gearbeitet werden, in dem die Phasentrennung schnell verläuft (die Dauer der Phasentrennung durchläuft ein Minimum in Abhängigkeit des molaren Verhältnisses von Anilin zu Formaldehyd).

Bis zum Erreichen des Zeitpunktes t₂ wird Anilin in solchen Mengen zudosiert, dass mindestens das 1,05-fache des in der Zielrezeptur vorgesehenen A/F_{Ziel}-Verhältnisses eingehalten wird. Im Anschluss daran wird der Anilin-Massenstrom m₁ so angepasst, dass das in der Zielrezeptur vorgesehene A/F_{Ziel}-Verhältnis eingehalten wird.

Bevorzugt werden die Einsatzstoffe Anilin und Formalin nach Durchmischung von oben als Reaktionsmischung in den Aminalreaktor eingetragen. Das im Aminalreaktor gebildete Reaktionsgemisch enthaltend das Aminal wird über einen Siphon dem sog. Aminalabscheider zugeführt. Über den Siphon wird der Stand im Aminalreaktor gehalten, und es wird ein Kontakt von Aminalreaktionslösung mit den Einsatzstoffen im Mischaggregat, wodurch eine Verblockung entstehen würde, verhindert. Es ist ebenfalls denkbar, wenn auch nicht bevorzugt, den Aminalreaktor mit den Einsatzstoffen von unten anzufahren und im Überlauf zu betreiben.

In **Schritt b)** erfolgt die Phasentrennung von organischer Aminalphase und wässriger Phase bei Temperaturen von 20 °C bis 120 °C, bevorzugt von 40 °C bis 110 °C, besonders bevorzugt von 60 °C bis 100 °C, bevorzugt bei Umgebungsdruck. Ferner kann die Phasentrennung auch bei leichtem Überdruck durchgeführt werden.

Die Umlagerung des Aminals in **Schritt c)** erfolgt in Gegenwart eines sauren Katalysators, üblicherweise einer starken Mineralsäure wie Salzsäure. Bevorzugt ist der Einsatz von Mineralsäure in einem molaren Verhältnis Mineralsäure zu Anilin von 0,001 bis 0,9, bevorzugt 0,05 bis 0,5. Es können natürlich auch feste, saure Katalysatoren, wie in der Literatur beschrieben, verwendet werden. Dabei kann Formaldehyd in eine Mischung von Anilin und saurem Katalysator gegeben und die Reaktionslösung durch stufenweises Erhitzen ausreagiert werden. Alternativ können auch Anilin und Formaldehyd zunächst vorreagiert werden und anschließend mit oder ohne vorhergehender Wasserabtrennung mit dem sauren Katalysator oder einem Gemisch von weiterem Anilin und saurem Katalysator versetzt werden, wonach die Reaktionslösung durch stufenweises Erhitzen ausreagiert wird. Diese Reaktion kann kontinuierlich nach einem der zahlreichen in der Literatur beschriebenen Verfahren ausgeführt werden (z. B. in EP 1 616 890 A1). Geeignete Salzsäurequalitäten sind z. B. in EP 1 652 835 A1 beschrieben.

Die Herstellung der rohen Di- und/oder Polyamine der Diphenylmethanreihe nach **Variante B)** lässt sich exemplarisch wie folgt zusammenfassen:
a) Kernvorgang des Schrittes B.I): Anilin und Säure werden in Abwesenheit von Formaldehyd zu einem Reaktionsgemisch umgesetzt, das das Aniliniumsalz der verwendeten Säure enthält, und
b) Kernvorgang des Schritts B.II): das Reaktionsgemisch aus Schritt a), das das Aniliniumsalz der verwendeten Säure enthält, wird mit Formaldehyd versetzt und zu MDA umgelagert.

Die Umsetzung von Anilin und Säure, bevorzugt Salzsäure, in **Schritt a)** kann nach einem Verfahren nach dem Stand der Technik durchgeführt werden. Die weitere Beschreibung erfolgt anhand des Beispiels wässriger Salzsäure, wobei auch andere Säuren zum Einsatz kommen können. Es werden normalerweise Anilin und eine wässrige Salzsäure bei Molverhältnissen von Anilin zu Säure im Bereich von 1,6 bis 100, bevorzugt 3,3 bis 20 umgesetzt. Diese Umsetzung kann in einem vorgelagerten Reaktor oder einer Mischstrecke erfolgen, wobei das Reaktionsgemisch fakultativ in einem Vorlagebehälter zwischengelagert werden kann. Optional kann diese Reaktion im gleichen Reaktor erfolgen, in dem auch die anschließende Umsetzung des Reaktionsgemisches von Anilin und Säure mit Formaldehyd stattfindet. Geeignete Anilinqualitäten sind z.B. beschrieben in EP 1 257 522 B1, EP 2 103 595 A1 und EP 1 813 598 B1. Geeignete Salzsäurequalitäten sind z.B. in EP 1 652 835 A1 beschrieben.

Zunächst kann "Einsatzanilin" im Reaktor bei Temperaturen von 10 °C bis 60 °C vorgelegt werden. Das Einsatzanilin setzt sich aus frischem Anilin und gegebenenfalls Anilin aus der MDA-Destillation (weiter unten näher beschrieben; siehe Schritt h)) und gegebenenfalls Anilin aus der Abwasseraufbereitung zusammen.

Dann erfolgt beispielsweise bei schon laufender Anilindosierung die Zugabe der Salzsäure in das vorgelegte Anilin, wobei für eine gute Durchmischung Sorge getragen wird. Diese gute Durchmischung kann durch Rühren mit einem Rührer erreicht werden oder auch durch ein im Kreis fahren (mittels Pumpen) der Reaktionsmischung oder durch eine Kombination aus Rühren und im Kreis fahren. Gegebenenfalls sollte die gesamte Anlage von Eduktströmen bis Produktabnahme betriebsbereit sein. Der Reaktionsapparat kann erforderlichenfalls mit einem internen oder externen Wärmeaustauscher ausgerüstet sein, um die entstehende Reaktionswärme abführen zu können. Alternativ kann auch das Einsatzanilin und/oder die Salzsäure entsprechend abgekühlt werden. Eine weitere Alternative bietet die Anwendung einer Siedekühlung zur Abführung der Reaktionswärme.

In **Schritt b)** erfolgt die Umsetzung des Anilinhydrochlorid-haltigen Reaktionsgemisches aus Schritt a) mit wässriger Formaldehydlösung. Dabei kann Formaldehyd in eine Mischung von Anilin und saurem Katalysator gegeben und die Reaktionslösung durch stufenweises Erhitzen ausreagiert werden, wie z. B. in EP 1 053 222 A1 beschrieben. Üblicherweise erfolgt die Umsetzung bei Temperaturen von 20 °C bis 150 °C.

Zweckmäßigerweise sind der Reaktor aus Schritt B.I) und der Reaktor aus Schritt B.II) voneinander verschieden. Es ist aber nicht ausgeschlossen, dass die Schritte B.I) und B.II) in demselben Reaktor ausgeführt werden. Diese Reaktion wird kontinuierlich ausgeführt.

Es werden bevorzugt technische Qualitäten an Formalin (wässrige Lösung von Formaldehyd) mit 30 Massen % bis 50 Massen % Formaldehyd in Wasser eingesetzt. Jedoch sind auch Formaldehydlösungen mit niedrigeren oder höheren Konzentrationen oder auch der Einsatz gasförmigen Formaldehyds denkbar.

Bis zum Erreichen des Zeitpunktes t₂ wird Formaldehyd in solchen Mengen zudosiert, dass mindestens das 1,05-fache des in der Zielrezeptur vorgesehenen A/F_{Ziel}-Verhältnisses eingehalten wird. Nach dem Anfahrprocedere werden die Anilin- und oder Formalinmengen so angepasst, dass das in der Zielrezeptur vorgesehene A/F_{Ziel}-Verhältnis eingehalten wird.

In **beiden Varianten A) und B)** wird ein rohes Reaktionsgemisch enthaltend Di- und Polyamine der Diphenylmethanreihe erhalten (in Variante A) in Schritt c) und in Variante B) in Schritt b)). Die Aufarbeitung dieses Reaktionsgemisches erfolgt unabhängig von der eingesetzten Variante A) oder B) bevorzug wie folgt:
d) Das Reaktionsgemisch enthaltend Di- und Polyamine der Diphenylmethanreihe aus Schritt A.c) bzw. B.b) wird neutralisiert, und
e) das neutralisierte Reaktionsgemisch enthaltend Di- und Polyamine der Diphenylmethanreihe wird in einem Trennbehälter in eine organische Phase enthaltend Di- und Polyamine der Diphenylmethanreihe und eine wässrige Phase aufgetrennt, und
f) die organische Phase enthaltend Di- und Polyamine der Diphenylmethanreihe wird in einem Waschbehälter mit Waschflüssigkeit weiter gereinigt, und
g) das so erhaltene Gemisch wird in einem Trennbehälter in eine organische Phase enthaltend Di- und Polyamine der Diphenylmethanreihe und eine wässrige Phase aufgetrennt, und
h) die gewaschene Di- und Polyamine der Diphenylmethanreihe enthaltende organische Phase wird destillativ von Wasser und Anilin befreit.

In **Schritt d)** wird das Reaktionsgemisch enthaltend die Di- und Polyamine der Diphenylmethanreihe ggf. unter Zusatz von Wasser und / oder Anilin neutralisiert. Nach dem Stand der Technik erfolgt die Neutralisation üblicherweise bei Temperaturen von beispielsweise 90 °C bis 100 °C ohne Zusatz weiterer Substanzen. Sie kann aber auch auf einem anderen Temperaturniveau erfolgen, um z.B. den Abbau von störenden Nebenprodukten zu beschleunigen.

Als Basen sind beispielsweise geeignet die Hydroxide der Alkali- und Erdalkalielemente. Vorzugsweise kommt wässrige NaOH zur Anwendung. Die zur Neutralisation eingesetzte Base wird bevorzugt in Mengen von größer als 100 %, besonders bevorzugt 105 % bis 120 % der für die Neutralisation des eingesetzten sauren Katalysators stöchiometrisch benötigten Menge eingesetzt (siehe EP 1 652 835 A1).

Anschließend wird in **Schritt e)** das neutralisierte Reaktionsgemisch enthaltend die Di- und Polyamine der Diphenylmethanreihe in eine organische Phase enthaltend Di- und Polyamine der Diphenylmethanreihe und eine wässrige Phase aufgetrennt. Dies kann durch die Zugabe von Anilin und/oder Wasser unterstützt werden. Wird die Phasentrennung durch Zugabe von Anilin und/oder Wasser unterstützt, so erfolgt deren Zugabe bevorzugt bereits unter intensivem Mischen in der Neutralisation. Dabei kann die Vermischung in Mischstrecken mit statischen Mischern, in Rührkesseln oder Rührkesselkaskaden oder aber in einer Kombination von Mischstrecken und Rührkessel erfolgen. Das neutralisierte und durch Zugabe von Anilin und/oder Wasser verdünnte Reaktionsgemisch wird anschließend bevorzugt einem Apparat zugeführt, der aufgrund seiner Konfiguration und/oder Einbauten besonders zur Auftrennung in eine organische Phase enthaltend MDA und eine wässrige Phase geeignet ist, bevorzugt Phasentrenn- oder Extraktionsvorrichtungen entsprechend dem Stand der Technik, wie sie beispielsweise in Mass-Transfer Operations, 3rd Edition, 1980, McGraw-Hill Book Co, S. 477 bis 541, oder Ullmann's Encyclopedia of Industrial Chemistry (Vol. 21, Liquid-Liquid Extraction, E. Müller et al., Seite 272-274, 2012 Wiley-VCH Verlag GmbH & Co. KGaA, Weinheim, DOI: 10.1002/14356007.b03_06.pub2) oder in Kirk-Othmer Encyclopedia of Chemical Technology (siehe "http://onlinelibrary.wiley.com/book/10.1002/0471238961", Published Online: 15 Juni 2007, Seite 22-23) beschrieben sind (Mixer-Settler-Kaskade oder Absetzbehälter).

In **Schritt f)** schließt sich eine Wäsche der organischen Phase mit Wasser und in Schritt g) eine neuerliche Abscheidung der Wasserphase zur Entfernung von Restgehalten an Salz (bevorzugt wie in DE-A-2549890, Seite 3 beschrieben) an.

In **Schritt h)** wird aus der in Schritt g) erhaltenen organischen Phase enthaltend Di- und Polyamine der Diphenylmethanreihe destillativ Wasser und Anilin abgetrennt, wie in EP 1 813 597 B1 beschrieben. Die in Schritt g) erhaltene organische Phase hat bevorzugt eine Zusammensetzung, bezogen auf das Gewicht des Gemisches, von 5-15 Gewichtsprozent Wasser, und, je nach Einsatzverhältnissen von Anilin und Formaldehyd, 5-90 Gewichtsprozent, bevorzugt 5 - 40 Gewichtsprozent Anilin und 5-90 Gewichtsprozent, bevorzugt 50 - 90 Gewichtsprozent Di- und Polyamine der Diphenylmethanreihe. Nach Austritt aus der Phasentrennung in Schritt g) hat die organische Phase enthaltend Di- und Polyamine der Diphenylmethanreihe üblicherweise eine Temperatur von 80 °C - 150 °C.

Die so erhaltenen Di- und Polyamine der Diphenylmethanreihe können nach den bekannten Methoden unter inerten Bedingungen mit Phosgen in einem organischen Lösungsmittel zu den entsprechenden Di- und Polyisocyanaten der Diphenylmethanreihe, dem MDI, umgesetzt werden. Dabei kann die Phosgenierung nach einem der aus dem Stand der Technik bekannten Verfahren durchgeführt werden (z.B. DE-A-844896 oder DE-A-19817691).

Werden die erfindungsgemäßen Bedingungen während des Anfahrens des Schrittes I) eingehalten, ergeben sich für beide Varianten A) und B) folgende Vorteile:
i) Vermeidung von Verblockungen und Ablagerungen in Reaktoren, in Kühleinrichtungen, Abscheidern und Kühlkreislaufpumpen und somit eine Vermeidung eines zweiten Anfahrvorganges, weil die Anlage zwecks Reinigung des Equipments nicht wieder abgefahren werden und geöffnet werden muss.
ii) Einsparung von Energie, weil wegen Entstehungen von Verblockungen und Ablagerungen und der daraus resultierenden Abstellung der Anlage zwecks Reinigung des Equipments der Anfahrvorgang nicht ein zweites Mal ausgeführt werden muss.
iii) Erhöhung der Produktivität der Anlage, weil wegen Wegfall der Reinigungszeiten zur Entfernung von Verblockungen und Ablagerungen sich die Reaktorlaufzeiten erhöhen.
iv) Vermeidung bzw. Verringerung von Niederschlägen, Anbackungen und Verstopfungen am Equipment (Reaktoren, Kühleinrichtungen, Abscheider und Kühlkreislaufpumpen) und damit einhergehend eine Verlängerung der Standzeit des Verfahrens.
v) Geringerer Abfall nach Reinigung des Equipments (hochmolekulare Feststoffe) und Einsparung von Verbrennungskosten.
vi) Vermeidung von nicht spezifikationsgerechter Ware, die durch mehrfaches schlechtes An- und Abfahren entstehen kann: solch eine qualitativ schlechte Abfahrware muss somit nicht mit qualitativ gutem MDA verschnitten werden oder sogar im schlimmsten Fall verbrannt werden.
vii) Eine bessere Phasentrennung zwischen wässriger und organischer Phase bedingt durch den Wegfall von die Phasentrennung negativ beeinflussenden hochmolekularen Verbindungen.

Die vorliegende Erfindung wird anhand der nachfolgenden Zeichnungen und Beispiele noch weiter erläutert, ohne jedoch darauf beschränkt zu sein.

Es zeigen:

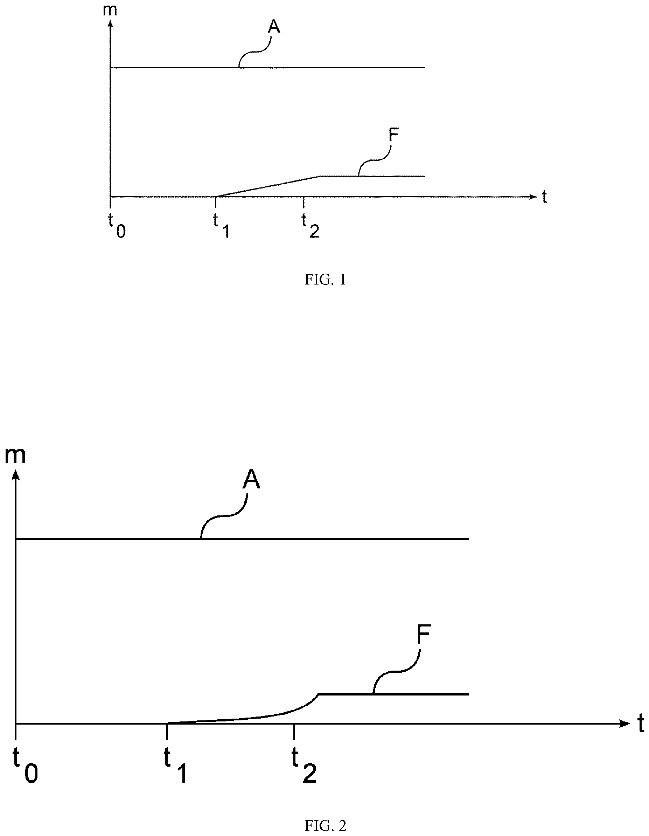

- FIG. 1-2: den zeitlichen Verlauf der Massenströme von Anilin und Formaldehyd im erfindungsgemäßen Verfahren.

FIG. 1 zeigt den zeitlichen Verlauf der Massenströme von Anilin und Formaldehyd in einer Ausführungsform der Variante A) des erfindungsgemäßen Verfahrens. Auf der x-Achse ist die Zeit t und auf der y-Achse sind Massenströme m aufgetragen. Zum Zeitpunkt t₀ ist Massenstrom in den in der Terminologie der vorliegenden Erfindung als Reaktor aus Schritt A.I) bezeichneten Aminal-Reaktor für Anilin (m₁, in der Figur mit "A" bezeichnet) bereits konstant und für Formaldehyd (m₂, in der Figur mit "F" bezeichnet) ist dieser null.

Nun wird beschlossen, dass die Produktion angefahren werden soll. Hierzu wird zum Zeitpunkt t₁ bei unveränderter Höhe des Massenstroms an Anilin der Massenstrom an Formaldehyd in den ersten Reaktor erhöht, bis er zum Zeitpunkt t₂ auf den Zielwert m_{2,Soll} angestiegen ist.

Man erkennt, dass zu jedem Zeitpunkt des Anfahrens der Reaktion der in den Aminal-Reaktor eingetragene Massenstrom des Anilins mindestens um so viel größer als der Massenstrom des Formaldehyds ist, dass das molare Verhältnis von Anilin zu Formaldehyd mindestens 2 beträgt.

In der Zeit zwischen t₁ und t₂ kann das im ersten Reaktor bereits befindliche Formaldehyd mit dem Anilin abreagieren. Letztendlich kann erreicht werden, dass im Aminal-Reaktor kein freies Formaldehyd vorliegt.

FIG. 2 zeigt analog zu FIG. 1 den zeitlichen Verlauf der Massenströme von Anilin und Formaldehyd in einer weiteren Ausführungsform der Variante A) des erfindungsgemäßen Verfahrens. Hier erfolgt die Zugabe des Formaldehyds ab dem Zeitpunkt t₁ nicht linear, sondern mit einem kurvenförmigen Verlaufsprofil.

### Beispiele: Allgemeine Bedingungen für die Herstellung von MDA bei eingefahrener Produktionsanlage (vor Beginn des Abfahrvorganges)

In einem kontinuierlichen Reaktionsprozess (Schritt a)) wurden 24,3 t/h Einsatzanilin (enthaltend 90 Massen-% Anilin) und 9,9 t/h 32 %ige wässrige Formaldehydlösung (molares Verhältnis Anilin zu Formalin 2,1 zu 1 vermischt und bei 90 °C und 1,4 bar absolut in einem gerührten Reaktionskessel zum Aminal umgesetzt. Der Reaktionskessel war mit einem Kühler mit Kühlkreislaufpumpe versehen. Das den Reaktionskessel verlassende Reaktionsgemisch wurde in einen Phasentrennapparat (Aminalabscheider) geführt (Schritt b)).

Nach der Phasentrennung zur Entfernung der wässrigen Phase wurde die organische Phase in einer Mischdüse mit 30 %iger wässriger Salzsäure versetzt (Protonierungsgrad 10 %, d.h., pro Mol Aminogruppen werden 0,1 Mol HCl zugegeben) und in den ersten Umlagerungsreaktor gefahren. Die Umlagerungsreaktion wurde in einer Reaktorkaskade bei 45 °C bis 165 °C durchgeführt (Schritt c)).

Nach vollständiger Reaktion wurde das erhaltene Reaktionsgemisch mit 32 %iger Natronlauge im molaren Verhältnis von 1,1 : 1 Natronlauge zu HCl versetzt und in einem Neutralisationsrührbehälter umgesetzt (Schritt d)). Die Temperatur betrug 115 °C. Der absolute Druck betrug 1,4 bar. Die neutralisierte Reaktionsmischung wurde anschließend in einem Neutralisationsabscheider in eine wässrige, untere Phase, die zu einem Abwassersammelbehälter gefühlt wird, und in eine organische Phase getrennt (Schritt e)).

Die organische, obere Phase wurde zur Wäsche geleitet und in einem gerührten Waschbehälter mit Kondensat und/oder Wasser aus dem Seitenstrom der Abwasserkolonne (Anilin/Wassergemisch) gewaschen (Schritt f)). Nach Abtrennung des Waschwassers in einem Waschwasserabscheider (Schritt g)) wurde das so erhaltene Roh-MDA durch Destillation von Wasser und Anilin befreit, wobei als Sumpfprodukt 17 t/h MDA anfielen (Schritt h)).

### Beispiel 1 (Vergleichsbeispiel): Anfahren der MDA-Anlage, wobei Formalin vorgelegt wird.

Nach Instandhaltungsarbeiten in der MDA-Anlage wurde der leere, gerührte Aminalreaktor mit 32 %iger wässriger Formaldehydlösung befüllt bis das Formaldehyd über das Siphon in den Aminalabscheider überlief. Wenn der Aminalreaktor so gefüllt war, lief das Formalin mit einer Last von 4,95 t/h in den gerührten Aminalreaktionskessel, was 50 % der Nennlast entsprach.

Nun wurde der Anilinweg freigegeben. Die Reaktion sprang sofort an und das Reaktionsgemisch wurde über einen Kühlwasserkreislauf auf 90 °C eingeregelt. Die Menge an Anilin, die während einer geplanten Anfahrzeit von 45 Minuten in den Aminalreaktor eingeleitet werden sollte, sollte stufenlos von 0 t/h auf 12,2 t/h Einsatzanilin hochgezogen werden. Nach 2 Minuten musste die Anlage abgestellt werden, weil der Aminalkessel, der Aminalkühler und der Aminalabscheider blockiert waren und die Aminal-Kühlkreislaufpumpe ebenfalls mit Feststoff geblockt war und stehenblieb.

### Beispiel 2 (Vergleichsbeispiel): Anfahren der MDA-Anlage, wobei Formalin- und Anilinzufuhr gleichzeitig in Betrieb genommen werden.

Nach Reparaturarbeiten in der MDA-Anlage wurde die Aminalreaktion angefahren, indem in den leeren Aminalreaktor gleichzeitig eine 32 %ige wässrige Formaldehydlösung und Einsatzanilin (enthaltend 90 Massen % Anilin) gefahren wurden. Die Menge der beiden Einsatzstoffe, die während der Anfahrzeit t von 45 Minuten in den gerührten Aminalreaktor eingeleitet wurden, wurde stufenlos von 0 t/h auf 4,95 t/h 32 %ige Formaldehydlösung und von 0 t/h auf 12,2 t/h Einsatzanilin (enthaltend 90 Massen % Anilin) hochgezogen.

Nach 2 Produktionstagen musste der Aminalreaktor außer Betrieb genommen werden, weil im Aminalabscheider ein extrem zähflüssiger, honigartiger Feststoff (unlösliche polymere Amine) ausgefallen war und der Ablauf des Aminalabscheiders verstopft war, was eine Reinigung erforderte.

### Beispiel 3 (erfindungsgemäß): Anfahren der MDA-Anlage, wobei Anilin vorgelegt wird.

Nach Instandhaltungsarbeiten im Aminalteil der MDA-Anlage wurde der leere Aminalreaktor mit Einsatzanilin (enthaltend 90 Massen-% Anilin) befüllt bis Anilin über das Siphon in den Aminalabscheider überlief. Wenn der Aminalreaktor so gefüllt war, lief das Einsatzanilin mit einer Last von 12,2 t/h in den gerührten Aminalreaktionskessel. Nun wurde der Formalinweg freigegeben. Die Reaktion sprang sofort an und das Reaktionsgemisch wurde über einen Kühlwasserkreislauf auf 90 °C eingeregelt. Der Druck im Aminalreaktor lag während der Anfahrphase bei 1,4 bar absolut.

Während einer Anfahrzeit von 45 Minuten wurde die Menge an 32 %iger wässriger Formaldehydlösung, die in den Aminalreaktor eingeleitet wurde, stufenlos von 0 t/h auf 4,95 t/h hochgezogen, was 50 % der Nennlast und einem A/F-Verhältnis von 2,1 zu 1 entsprach. Anschließend wurde die Reaktionsmischung aus dem Aminalreaktor in einen Phasentrennapparat gefahren, in dem das Reaktionswasser aus der Aminalreaktion abgetrennt wurde.

Die verbliebene organische Phase wurde nun in den ersten Umlagerungstank gepumpt, wobei gleichzeitig eine 30 %ige wässrige Salzsäure, entsprechend einem Protonierungsgrad von 10 % (d. h., pro Mol Aminogruppen werden 0,1 Mol HCl zugegeben), über eine Mischdüse in den Einlauf der organischen Phase (Aminal) in den ersten Umlagerungstank eindosiert wurde. Die Umlagerungsreaktion fand bei 50 °C bis 150 °C in einer Reaktorkaskade statt (Schritt c)). Nach vollständiger Reaktion wurde das erhaltene Reaktionsgemisch, wie in den allgemeinen Herstellungsbedingungen von MDA beschrieben, aufgearbeitet.

Bei erfindungsgemäßer Vorgehensweise wurde während der Anfahrphase eine Verblockung im Aminalkessel, im Aminalkühler, im Aminalabscheider und auch ein Festsitzen der Aminal-Kühlkreislaufpumpe durch Feststoffablagerungen vermieden. Der Aminalkessel konnte über einen langen Produktionszyklus von mehreren Monaten betrieben werden. Die Entstehung von unerwünschten Nebenprodukten wie unlösliche polymere Amine etc. war signifikant reduziert, und ein späteres Verschneiden der Anfahrware mit reinem MDA oder im schlimmsten Fall die Verbrennung der Anfahrware konnte unterbleiben.

### Beispiel 4 (erfindungsgemäß): Anfahren der MDA-Anlage, wobei Anilin in der Kondensation und Anilinhydrochlorid in der Umlagerung vorgelegt werden.

Nach einer Abstellung der MDA-Anlage wurde der leere Aminalreaktor mit Einsatzanilin (enthaltend 90 Massen % Anilin) befüllt bis Anilin über das Siphon in den Aminalabscheider überlief. Wenn der Aminalreaktor so gefüllt war, lief das Einsatzanilin mit einer Last von 12,2 t/h in den gerührten Aminalreaktionskessel. Nun wurde der Formalinweg freigegeben.

Die Reaktion sprang sofort an und das Reaktionsgemisch wurde auf 90 °C eingeregelt. Der Druck im Aminalreaktor lag während der Anfahrphase bei 1,4 bar absolut. Während einer Anfahrzeit von 45 Minuten wurde die Menge an 32 %iger Formaldehydlösung, die in den Aminalreaktor eingeleitet wurde, stufenlos von 0 t/h auf 4,95 t/h hochgezogen, was 50 % der Nennlast entsprach.

Anschließend wurde die Reaktionsmischung aus dem Aminalreaktor in einen Phasentrennapparat gefahren, in dem das Reaktionswasser aus der Aminalreaktion abgetrennt wurde. Die verbliebene organische Phase wurde nun in den ersten Umlagerungsreaktor gepumpt, der bei einem Füllstand von 60 % mit Anilinhydrochlorid gefüllt war. Gleichzeitig wurde eine 30 %ige wässrige Salzsäure, entsprechend einem Protonierungsgrad von 10 % (d. h., pro Mol Aminogruppen werden 0,1 Mol HCl zugegeben) über eine Mischdüse in den Einlauf des Aminals in den ersten Umlagerungstank eindosiert. Die Umlagerungsreaktion wurde bei 50 °C bis 165 °C in einer Reaktorkaskade durchgeführt (Schritt c)). Nach vollständiger Reaktion wurde das erhaltene Reaktionsgemisch, wie in den allgemeinen Herstellungsbedingungen von MDA beschrieben, aufgearbeitet.

Bei erfindungsgemäßer Vorgehensweise wurde während der Anfahrphase eine Verblockung im Aminalkessel, im Aminalkühler, im Aminalabscheider und auch ein Festsitzen der Aminal-Kühlkreislaufpumpe durch Feststoffablagerungen vermieden. Der Aminalkessel konnte über einen langen Produktionszyklus von mehreren Monaten betrieben werden. Zusätzlich traten auch im ersten Umlagerungsreaktor keinerlei Probleme mit Ablagerungen auf. Die Entstehung von unerwünschten Nebenprodukten wie unlösliche polymere Amine etc. war signifikant reduziert, und ein späteres Verschneiden der Anfahrware mit reinem MDA oder im schlimmsten Fall die Verbrennung der Anfahrware konnte unterbleiben.

## Patentansprüche

1. Verfahren zur Herstellung von Di- und Polyaminen der Diphenylmethanreihe (MDA) durch Umsetzung von Anilin (1) und Formaldehyd (2) bei einem angestrebten molaren Verhältnis von Anilin (1) zu Formaldehyd (2) von A/F_{Ziel}, umfassend die Schritte:
Entweder gemäß einer Variante A)
A.I) Reaktion von Anilin (1) und Formaldehyd (2) in einem Reaktor in Abwesenheit eines sauren Katalysators (3) unter Bildung eines Aminals, wobei Anilin (1) und Formaldehyd (2) in den Reaktor eingetragen werden, und anschließende Trennung des erhaltenen Reaktionsgemisches in eine wässrige und eine organische, Aminal enthaltende Phase;
A.II) Reaktion zumindest eines Teils der in Schritt A.I) erhaltenen organischen, Aminal enthaltenden Phase in einem Reaktor mit Säure (3), wobei das Aminal zu Di- und Polyaminen der Diphenylmethanreihe reagiert;
wobei die Schritte A.I) und A.II) kontinuierlich durchgeführt werden und wobei für die Aufnahme des Verfahrens und/oder die Wiederaufnahme des Verfahrens nach einer Unterbrechung zumindest des Schritts A.I) die folgenden Schritte durchlaufen werden:
A.I.1) Eintragen von Anilin (1), beginnend bei dem Zeitpunkt t₀, in den Reaktor aus Schritt A.I) mit einem Massenstrom m₁;
A.I.2) Eintragen von Formaldehyd (2), beginnend bei einem Zeitpunkt t₁, in den Reaktor aus Schritt A.I) ausgehend von einem Massenstrom m₂ = 0 beim Zeitpunkt t₁ bis zu einem Massenstrom m₂ = m_{2,Soll} beim Zeitpunkt t₂, wobei t₂ > t₁ > t₀ und wobei die Zeitspanne t₂ - t₁ bei > 0 Stunden und < 20 Stunden liegt, wobei der Zeitpunkt t₁ so gewählt wird, dass zu diesem Zeitpunkt der Reaktor zu 10 % bis 99 % seines maximalen Fassungsvermögens mit Anilin befüllt ist;
A.II.1) im Falle der Unterbrechung auch des Schritts A.II), Eintragen von Säure (3) in den Reaktor aus Schritt A.II) spätestens wenn, sobald oder nachdem erstmalig organische, Aminal enthaltende Phase in den Reaktor aus Schritt A.II) eingetragen wird;
wobei das Eintragen von Anilin (1) in Schritt A.I.1) und das Eintragen von Formaldehyd (2) in Schritt A.I.2) derart geschehen, dass im Zeitraum von t₁ bis t₂ das momentane molare Verhältnis von
dem Reaktor aus Schritt A.I) insgesamt zugegebenem Anilin (1)
zu
dem Reaktor aus Schritt A.I) insgesamt zugegebenem Formaldehyd (2),
A/F_{mom.}, stets ≥ 2 und ≥ 1,05 · A/F_{Ziel} beträgt,
wobei nach Erreichen des angestrebten Formaldehyd-Massenstroms m_{2,Soll} zum Zeitpunkt t₂ das molare Verhältnis von Anilin (1) zu Formaldehyd (2) auf A/F_{Ziel} eingestellt wird;
oder gemäß einer Variante B)
B.I) Reaktion von Anilin (1) und Säure (3) in einem Reaktor unter Bildung eines Reaktionsgemisches, das das Aniliniumsalz der verwendeten Säure (3) enthält;
B.II) Reaktion zumindest eines Teils des in Schritt B.I) erhaltenen Reaktionsgemisches mit Formaldehyd (2) in einem Reaktor, wobei Di- und Polyamine der Diphenylmethanreihe entstehen;
wobei die Schritte B.I) und B.II) kontinuierlich durchgeführt werden und wobei für die Aufnahme des Verfahrens und/oder die Wiederaufnahme des Verfahrens nach einer Unterbrechung der Schritte B.I) und B.II) folgende Schritte durchlaufen werden:
B.I.1) Eintragen von Anilin (1), beginnend bei dem Zeitpunkt t₀, in den Reaktor aus Schritt B.I) mit einem Anilin-Massenstrom m₁;
B.I.2) Eintragen von Säure (3) vor, gleichzeitig mit oder nach dem Eintragen von Anilin (1); B.II.1) Eintragen von Formaldehyd (2) in den Reaktor aus Schritt B.II), optional gemeinsam mit weiterem Anilin (1), optional gemeinsam mit weiterer Säure (3), beginnend bei einem Zeitpunkt t₁, ausgehend von einem Massenstrom m₂ = 0 beim Zeitpunkt t₁ bis zu einem Massenstrom m₂ = m_{2,Soll} beim Zeitpunkt t₂, wobei t₂ > t₁ > t₀ und wobei die Zeitspanne t₂ - t₁ bei > 0 Stunden und < 20 Stunden liegt, wobei der Zeitpunkt t₁ so gewählt wird, dass zu diesem Zeitpunkt der Reaktor zu 10 % bis 99 % seines maximalen Fassungsvermögens mit Anilin befüllt ist;
wobei das Eintragen von Anilin (1) in Schritt B.I.1) sowie ggf. in Schritt B.II.1) und das Eintragen von Formaldehyd (2) in Schritt B.II.1) derart geschehen, dass im Zeitraum von t₁ bis t₂ das momentane molare Verhältnis von
dem Reaktor aus Schritt B.I) insgesamt zugegebenem Anilin (1) und, sofern vorhanden, dem Reaktor aus Schritt B.II) insgesamt zugegebenem Anilin (1)
zu
dem Reaktor aus Schritt B.II) insgesamt zugegebenem Formaldehyd (2),
A/F_{mom.}, stets ≥ 2 und ≥ 1,05 · A/F_{Ziel} beträgt,
wobei nach Erreichen des angestrebten Formaldehyd-Massenstroms m_{2,Soll} zum Zeitpunkt t₂ das molare Verhältnis von Anilin (1) zu Formaldehyd (2) auf A/F_{Ziel} eingestellt wird.

2. Verfahren gemäß Anspruch 1, wobei in beiden Varianten A/F_{Ziel} einen Wert von 1,5 bis 20, bevorzugt von 1,5 bis 10 und besonders bevorzugt von 1,5 bis 6 aufweist.

3. Verfahren gemäß einem der Ansprüche 1 oder 2, wobei in beiden Varianten die Zeitspanne t₂ - t₁ bei > 0 Stunden und < 10 Stunden liegt.

4. Verfahren gemäß einem oder mehreren der vorhergehenden Ansprüche, wobei in beiden Varianten die Zeitspanne t₂ - t₁ bei > 0 Stunden und < 5 Stunden liegt.

5. Verfahren gemäß einem oder mehreren der vorhergehenden Ansprüche, wobei in beiden Varianten der Massenstrom m₁ ≥ 1000 kg/Stunde beträgt.

6. Verfahren gemäß einem oder mehreren der vorhergehenden Ansprüche, wobei in beiden Varianten der Massenstrom m_{2,Soll} ≥ 300 kg/Stunde beträgt.

7. Verfahren gemäß einem oder mehreren der vorhergehenden Ansprüche, wobei in beiden Varianten der Zeitpunkt t₁ so gewählt wird, dass zu diesem Zeitpunkt der jeweilige Reaktor zu 10 % bis 90 % seines maximalen Fassungsvermögens mit Anilin befüllt ist.

8. Verfahren gemäß einem oder mehreren der vorhergehenden Ansprüche, wobei in beiden Varianten der Zeitpunkt t₁ so gewählt wird, dass zu diesem Zeitpunkt der jeweilige Reaktor zu 20 % bis 80 % seines maximalen Fassungsvermögens mit Anilin befüllt ist.

## Claims

1. Process for preparing diamines and polyamines of the diphenylmethane series (MDA) by reaction of aniline (1) and formaldehyde (2) at a desired molar ratio of aniline (1) to formaldehyde (2) of A/F_{target}, which comprises the steps:
either according to a variant A)
A.I) reaction of aniline (1) and formaldehyde (2) in the absence of an acid catalyst (3) in a reactor to form an aminal, with aniline (1) and formaldehyde (2) being introduced into the reactor, and subsequent separation of the reaction mixture obtained into an aqueous phase and an organic, aminal-containing phase;
A.II) reaction of at least part of the organic, aminal-containing phase obtained in step A.I) with acid (3) in a reactor, with the aminal reacting to form diamines and polyamines of the diphenylmethane series;
wherein steps A.I) and A.II) are performed continuously and wherein the following steps are carried out for start-up of the process and/or resumption of the process after an interruption of at least step A.I):
A.I.1) introduction of aniline (1) into the reactor of step A.I) at a mass flow rate m₁ commencing at the point in time t₀;
A.I.2) introduction of formaldehyde (2) into the reactor of step A.I), commencing at a point in time t₁, starting from a mass flow rate m₂ = 0 at the point in time t₁ to a mass flow rate m₂ = m_{2,intended} at the point in time t₂, where t₂ > t₁ > t₀, and wherein the period of time t₂ - t₁ is > 0 hours and < 20 hours, wherein the point in time t₁ is selected so that at this point in time the reactor is full of aniline to an extent of from 10% to 99% of its maximum capacity;
A.II.1) in the case of interruption of the step A.II) too, introduction of acid (3) into the reactor of step
A.II) at the latest when, as soon as or after, organic, aminal-containing phase is introduced for the first time into the reactor of step A.II);
where the introduction of aniline (1) in step A.I.1) and the introduction of formaldehyde (2) in step A.I.2) occur in such a way that the instantaneous molar ratio of the total aniline (1) introduced into the reactor of step A.I)
to
the total formaldehyde (2) introduced into the reactor of step A.I),
A/F_{inst.}, is always ≥ 2 and ≥ 1.05 · A/F_{target} during the period of time from t₁ to t₂,
wherein, after the desired formaldehyde mass flow rate m_{2,intended} has been reached at the point in time t₂, the molar ratio of aniline (1) to formaldehyde (2) is set to A/F_{target},
or according to a variant B),
B.I) reaction of aniline (1) and acid (3) in a reactor to form a reaction mixture containing the anilinium salt of the acid (3) used;
B.II) reaction of at least part of the reaction mixture obtained in step B.I) with formaldehyde (2) in a reactor, forming diamines and polyamines of the diphenylmethane series;
wherein steps B.I) and B.II) are performed continuously and wherein the following steps are carried out for the start-up of the process and/or the resumption of the process after an interruption of the steps B.I) and B.II) :
B.I.1) introduction of aniline (1) into the reactor of step B.I) at an aniline mass flow rate m₁ commencing at the point in time t₀;
B.I.2) introduction of acid (3) before, simultaneously with or after introduction of aniline (1);
B.II.1) introduction of formaldehyde (2) into the reactor of step B.II), optionally together with further aniline (1), optionally together with further acid (3), commencing at a point in time t₁, proceeding from a mass flow rate m₂ = 0 at the point in time t₁ to a mass flow rate m₂ = m_{2,intended} at the point in time t₂, where t₂ > t₁ > t₀ and wherein the period of time t₂ - t₁ is > 0 hours and < 20 hours, wherein the point in time t₁ is selected so that at this point in time the reactor is full of aniline to an extent of from 10% to 99% of its maximum capacity; where the introduction of aniline (1) in step B.I.1) and optionally in step B.II.1) and the introduction of formaldehyde (2) in step B.II.1) occur in such a way that the instantaneous molar ratio of
the total aniline (1) introduced into the reactor of step B.I) and, if present, the total aniline (1) introduced into the reactor of step B.II)
to
the total formaldehyde (2) introduced into the reactor of step B.II),
A/F_{inst.}, is always ≥ 2 and ≥ 1.05 · A/F_{target} during the period of time from t₁ to t₂,
wherein, after the desired formaldehyde mass flow rate m_{2,intended} has been reached at the point in time t₂, the molar ratio of aniline (1) to formaldehyde (2) is set to A/F_{target}.

2. Process according to Claim 1, wherein A/F_{target} has a value of from 1.5 to 20, preferably from 1.5 to 10 and particularly preferably from 1.5 to 6, in both variants.

3. Process according to either of Claims 1 and 2, wherein the period of time t₂ - t₁ is > 0 hours and < 10 hours in both variants.

4. Process according to one or more of the preceding claims, wherein the period of time t₂ - t₁ is > 0 hours and < 5 hours in both variants.

5. Process according to one or more of the preceding claims, wherein the mass flow rate m₁ is ≥ 1000 kg/hour in both variants.

6. Process according to one or more of the preceding claims, wherein the mass flow rate m_{2, intended} is ≥ 300 kg/hour in both variants.

7. Process according to one or more of the preceding claims, wherein, in both variants, the point in time t₁ is selected so that at this point in time the respective reactor is full of aniline to an extent of from 10% to 90% of its maximum capacity.

8. Process according to one or more of the preceding claims, wherein, in both variants, the point in time t₁ is selected so that at this point in time the respective reactor is full of aniline to an extent of from 20% to 80% of its maximum capacity.

## Revendications

1. Procédé de préparation de di- et de polyamines de la série du diphénylméthane (MDA) par réaction d'aniline (1) et de formaldéhyde (2), pour un rapport en moles recherché de l'aniline (1) au formaldéhyde (2) A/F_{Ziel}, comprenant les étapes :
selon une variante A)
A.I) Réaction de l'aniline (1) et du formaldéhyde (2) dans un réacteur en l'absence de catalyseurs acides (3) avec formation d'un aminal, l'aniline (1) et le formaldéhyde (2) étant introduits dans le réacteur, puis séparation du mélange réactionnel obtenu en une phase aqueuse et une phase organique contenant l'aminal ;
A.II) Réaction d'au moins une partie de la phase organique obtenue dans l'étape A.I), contenant l'aminal,
dans un réacteur avec un acide (3), l'aminal réagissant pour donner des di- et des polyamines de la série du diphénylméthane ;
dans lequel les étapes A.I) et A.II) sont mises en œuvre en continu, et, pour le lancement du procédé et/ou la reprise du procédé après une interruption au moins de l'étape A.I), on procède aux étapes suivantes :
A.I.1) Introduction de l'aniline (1), à partir de l'instant t₀, dans le réacteur de l'étape A.I), selon un débit massique m₁ ;
A.I.2) Introduction du formaldéhyde (2), à partir de l'instant t₁, dans le réacteur de l'étape A.I), en partant d'un débit massique m₂ = 0 à l'instant t₁ pour arriver à un débit massique m₂ = m_{2,Soll} à l'instant t₂, avec t₂ > t₁ > t₀, et le laps de temps t₂ - t₁ étant > 0 heure et < 20 heures, l'instant t₁ étant choisi de telle sorte que, à cet instant-là, le réacteur soit rempli d'aniline à raison de 10 % à 99 % de sa capacité maximale ;
A.II.1) dans le cas de l'interruption également de l'étape A.II), introduction d'un acide (3) dans le réacteur de l'étape A.II) au plus tard au moment où la phase organique contenant l'aminal est introduite dans le réacteur de l'étape A.II), ou dès cette introduction, ou après cette première introduction ;
dans lequel l'introduction de l'aniline (1) dans l'étape A.I.1) et l'introduction du formaldéhyde (2) dans l'étape A.I.2) sont réalisées de façon que, dans le laps de temps de t₁ à t₂, le rapport instantané en moles de l'aniline (1) globalement introduite dans le réacteur de l'étape A.I) au formaldéhyde (2) globalement introduit dans le réacteur de l'étape A.I), soit
A/F_{mom.} toujours ≥ 2 et ≥ 1,05 • A/F_{Ziel},
dans lequel, après que l'on a atteint à l'instant t₂ le débit massique recherché de formaldéhyde m_{2,Soll}, le rapport en moles de l'aniline (1) au formaldéhyde (2) est ajusté à A/F_{Ziel} ;
ou selon une variante B)
B.I) Réaction de l'aniline (1) et de l'acide (3) dans un réacteur avec formation d'un mélange réactionnel qui contient le sel d'anilinium de l'acide utilisé (3) ;
B.II) Réaction d'au moins une partie du mélange réactionnel obtenu dans l'étape B.I) avec du formaldéhyde (2) dans un réacteur, avec formation de di- et de polyamines de la série du diphénylméthane ;
dans lequel les étapes B.I) et B.II) sont mises en œuvre en continu, et, pour le lancement du procédé et/ou la reprise du procédé après une interruption des étapes B.I) et B.II), on procède aux étapes suivantes :
B.I.1) Introduction de l'aniline (1), à partir de l'instant t₀, dans le réacteur de l'étape B.I) avec un débit massique d'aniline m₁ ;
B.I.2) Introduction de l'acide (3) avant ou après l'introduction de l'aniline (1), ou en même temps que cette introduction ;
B.II.1) Introduction du formaldéhyde (2) dans le réacteur de l'étape B.II), éventuellement en même temps qu'une quantité supplémentaire de l'aniline (1), éventuellement en même temps qu'une quantité supplémentaire de l'acide (3), en commençant à l'instant t₁, à partir d'un débit massique m₂ = 0 à l'instant t₁ jusqu'à un débit massique m₂ = m_{2,Soll} à l'instant t₂, avec t₂ > t₁ > t₀, et le laps de temps t₂ - t₁ étant > 0 heure et < 20 heures, l'instant t₁ étant choisi de façon que, à cet instant-là, le réacteur soit rempli d'aniline à raison de 10 % à 99 % de sa capacité maximale ;
dans lequel l'introduction de l'aniline (1) dans l'étape B.I.1), ainsi qu'éventuellement dans l'étape B.II.1), et l'introduction du formaldéhyde (2) dans l'étape B.II.1) sont réalisées de façon que, dans le laps de temps de t₁ à t₂, le rapport instantané en moles
de l'aniline (1) globalement introduite dans le réacteur de l'étape B.I) et, si elle est présente, de l'aniline (1) globalement introduite dans le réacteur de l'étape B.II) au formaldéhyde (2) globalement introduit dans le réacteur de l'étape B.II),
A/F_{mom.} soit toujours ≥ 2 et ≥ 1,05 • A/F_{Ziel},
dans lequel, après que l'on a atteint le débit massique recherché du formaldéhyde m_{2,Soll} à l'instant t₂, on ajuste le rapport en moles de l'aniline (1) au formaldéhyde (2) à A/F_{Ziel}.

2. Procédé selon la revendication 1, dans lequel, dans les deux variantes, A/F_{Ziel} présente une valeur de 1,5 à 20, de préférence de 1,5 à 10 et d'une manière particulièrement préférée de 1,5 à 6.

3. Procédé selon l'une des revendications 1 ou 2, dans lequel, dans les deux variantes, le laps de temps t₂ - t₁ est > 0 heure et < 10 heures.

4. Procédé selon l'une ou plusieurs des revendications précédentes, dans lequel, dans les deux variantes, le laps de temps t₂ - t₁ est > 0 heure et < 5 heures.

5. Procédé selon l'une ou plusieurs des revendications précédentes, dans lequel, dans les deux variantes, le débit massique m₁ ≥ 1 000 kg/heure.

6. Procédé selon l'une ou plusieurs des revendications précédentes, dans lequel, dans les deux variantes, le débit massique m_{2,Soll} ≥ 300 kg/heure.

7. Procédé selon l'une ou plusieurs des revendications précédentes, dans lequel, dans les deux variantes, l'instant t₁ est choisi de façon que, à cet instant-là, le réacteur considéré soit rempli d'aniline à raison de 10 % à 90 % de sa capacité maximale.

8. Procédé selon l'une ou plusieurs des revendications précédentes, dans lequel, dans les deux variantes, l'instant t₁ est choisi de façon que, à cet instant-là, le réacteur considéré soit rempli d'aniline à raison de 20 % à 80 % de sa capacité maximale.
